# EUROPEAN PATENT APPLICATION

(11) **EP 4 495 229 A1**
(43) Date of publication of application: **22.01.2025**
(21) Application number: 23771060.3
(22) Date of filing: 14.03.2023
(51) Int. Cl.: C12N 5/0735, C12N 5/074

(54) **MEDIUM COMPOSITION FOR CULTURING PORCINE PLURIPOTENT STEM CELLS**

(30) Priority: 14.03.2022 KR 20220031249; 13.03.2023 KR 20230032368
(71) Applicant: Space F Corporation, Hwaseong-si, Gyeonggi-do 18471 (KR)
(72) Inventor: LEE, Chang-Kyu, Seoul 06547 (KR); CHOI, Kwang-Hwan, Seoul 08774 (KR); LEE, Dong-Kyung, Hanam-si, Gyeonggi-do 13011 (KR)
(74) Representative: dompatent von Kreisler Selting Werner - Partnerschaft von Patent- und Rechtsanwälten mbB
(86) International application number: PCT/KR2023/003393
(87) International publication number: WO 2023/177181

(57) **Abstract**

The present invention relates to a medium composition for culturing porcine pluripotent stem cells, and the medium composition for culture according to the present invention may maintain the pluropotency and stemnness of porcine pluripotent stem cells *in vitro* for a long period of time and allows stem cells to be cultured in a feeder cell-free manner.

## Description

### [Technical Field]

This application claims the benefits of priority based on Korean Patent Application No. 10-2022-0031249 filed on March 14, 2022, and Korean Patent Application No. 10-2023-0032368 filed on March 13, 2023, and the entire contents of which are incorporated herein as a part of the present specification.

The present invention relates to a medium composition for culturing porcine pluripotent stem cells.

### [Background Art]

The acquisition and maintenance of pluripotency in stem cells is achieved or maintained by activation of relevant genes through external factors such as metabolites, signaling molecules and extracellular matrix (ECM). Metabolites such as carbohydrates, amino acids and lipids are basic components necessary for culturing cells *in vitro* and regulate the physiological characteristics and pluripotency of stem cells. Various signaling molecules, including cytokines and hormones, play an important role in triggering the activation of cell signaling systems to support pluripotency and inhibit differentiation of stem cells. Reportedly, pluripotency changes dynamically during embryonic development and constitutes a continuum consisting of several stages, such as naive, formative and primed states. It has been demonstrated that pluripotency at each stage is regulated by different combinations of signaling molecules, and numerous efforts are underway to discover novel molecules to maintain pluripotency.

The extracellular matrix is composed of structural proteins such as collagen, laminin and fibronectin, and provides a physical environment for cell adhesion and survival. Cells recognize extracellular matrix proteins through integrin receptors to regulate cell proliferation, survival and aging. Feeder cells made from mouse fibroblasts have been used for a long time to culture pluripotent stem cells *in vitro.* The feeder cells are known to maintain the pluripotency of pluripotent stem cells by providing paracrine factors and an adhesive ECM surface.

Although the use of feeder cells is advantageous for culturing pluripotent stem cells *in vitro,* several issues have been raised regarding xenobiotic contamination and cell-to-cell variation. Thus, numerous studies have been attempted to replace feeder cells derived from mouse fibroblasts. First, conditioned media of mouse embryonic fibroblasts are used instead of feeder cells, or new culture conditions have been developed using allogenic cells such as foreskin fibroblasts, endometrium-derived cells and mesenchymal stem cells in human embryonic stem cell research.

However, some culture conditions could not support the undifferentiated state of pluripotent stem cells depending on the cell origin, but it was confirmed through several proteome analyses that pluripotency-supportive feeder cells highly express various cytokines, including FGF2, TGFβ1, activin A and WNT. These molecules have been proven to improve the expression of pluripotency-related genes in pluripotent stem cells even without feeder cells.

In addition, the possibility of replacing the extracellular matrix provided by feeder cells with Matrigel, or single components such as collagen, laminin and fibronectin is being studied.

Porcine pluripotent stem cells such as embryonic stem cells are considered an applicable cell source for research in agricultural biotechnology and comparative developmental biology. However, since pigs have unique molecular biological characteristics compared to mouse and human embryos in the early preimplantation embryonic development when pluripotency is established, many researchers have failed to establish embryonic stem cells in pigs. In previous inventions, our research team discovered various growth factors involved in porcine pluripotency and developed a chemically defined medium containing a new pig-specific combination of growth factors, which was used together with mouse fibroblast-derived feeder cells to establish porcine embryonic stem cells with *in vivo* differentiation potential for the first time in the world. However, it was confirmed that when porcine embryonic stem cells were cultured in the previously developed culture medium composition in an environment without feeder cells, they differentiated without maintaining pluripotency.

Feeder cells made from mouse embryonic fibroblasts produce a variety of cytokines, including fibroblast growth factor 2 (FGF2), transforming growth factor β1 (TGFβ1), activin A, WNT, oncostatin M and interleukin-6 (IL-6). Among them, FGF2 has been used as a key element in maintaining the pluripotency of human embryonic stem cells, and activin A, which is the standard for feeder cell quality, is abundant in conditioned media in which mouse embryonic fibroblasts are cultured. In fact, medium containing activin A and WNT helped maintain pluripotency of human embryonic stem cells in the absence of feeder cells. However, unlike human pluripotent stem cells, FGF2, activin A or TGFβ1 and WNT antagonists failed to culture porcine embryonic stem cells *in vitro* under similar conditions in an environment without feeder cells. This demonstrates that there is a need to establish new culture conditions containing additional growth factors or signaling molecules in order to culture porcine embryonic stem cells *in vitro* without feeder cells.

Under this background, as a result of studying the composition for culturing porcine pluripotent stem cells in various aspects, the present inventors have completed the present invention by confirming that feeder cell-free culture of porcine pluripotent stem cells was possible when using the culture composition of the present invention.

### [Prior Art Documents]

### [Patent Documents]

(Patent Document 1) Korean Patent No. 10-2142400 (Registration Date: August 3, 2020)

### [Non-Patent Documents]

(Non-Patent Document 1) Choi KH, Lee DK, Oh JN, Kim SH, Lee M, Woo SH, Kim DY, Lee CK. Pluripotent pig embryonic stem cell lines originating from in vitro-fertilized and parthenogenetic embryos. Stem Cell Res. 2020 Dec;49:102093.
(Non-Patent Document 2) Choi KH, Lee DK, Oh JN, Kim SH, Lee M, Kim SW, Lee CK. Transcriptome profiling of pluripotent pig embryonic stem cells originating from uni- and biparental embryos. BMC Res Notes. 2020 Mar 11;13(1):144.
(Non-Patent Document 3) Choi KH, Lee DK, Kim SW, Woo SH, Kim DY, Lee CK. Chemically Defined Media Can Maintain Pig Pluripotency Network In Vitro. Stem Cell Reports. 2019 Jul 9;13 (1) :221-234.

### [Disclosure]

### [Technical Problem]

It is an object of the present invention to provide a medium composition for culturing porcine pluripotent stem cells.

It is another object of the present invention to provide a feeder cell-free method of culturing porcine pluripotent stem cells using the medium composition for culturing porcine pluripotent stem cells.

### [Technical Solution]

In order to achieve the above objects, the present invention provides a medium composition for culturing porcine pluripotent stem cells comprising LDN-193189.

According to one embodiment of the present invention, the medium composition for culturing porcine pluripotent stem cells may further comprise any one or more components selected from FGF2, activin A, IWR-1 and CHIR99021.

According to one embodiment of the present invention, the present invention provides a method of culturing porcine pluripotent stem cells using the medium composition for culturing porcine pluripotent stem cells comprising LDN-193189.

According to one embodiment of the present invention, the present invention provides a composition for addition to a porcine pluripotent stem cell culture medium comprising LDN-193189.

### [Advantageous Effects]

The medium composition for culturing porcine pluripotent stem cells according to the present invention may maintain pluripotency/stemness even without feeder cells, thereby solving problems that may arise due to feeder cell-dependent culture methods.

### [Description of Drawings]

Figure 1 shows cell morphology when porcine embryonic stem cells cultured without feeder cells were treated with LDN-193189 at different concentrations (100x magnification).

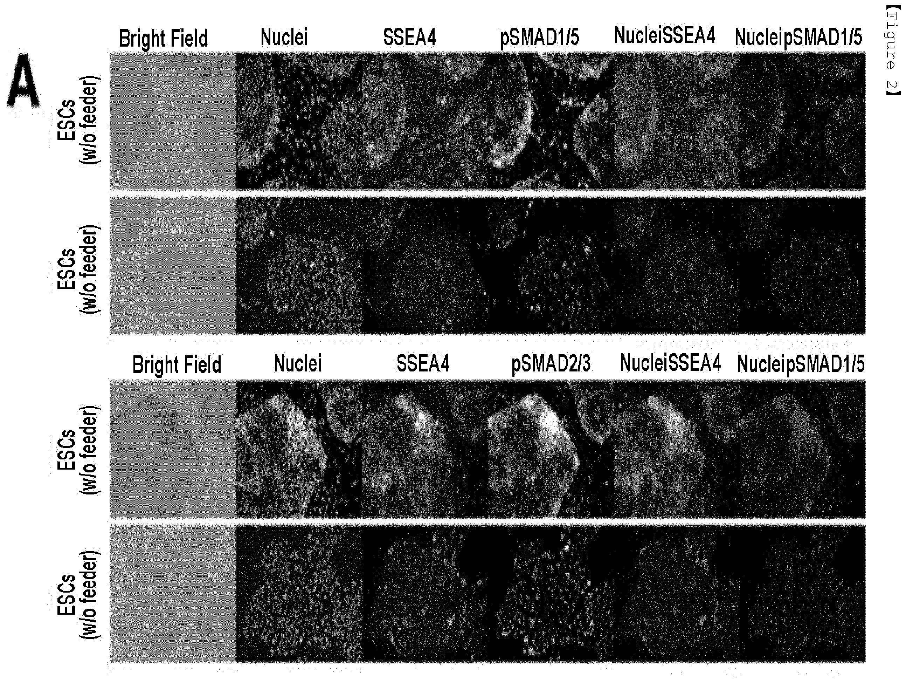

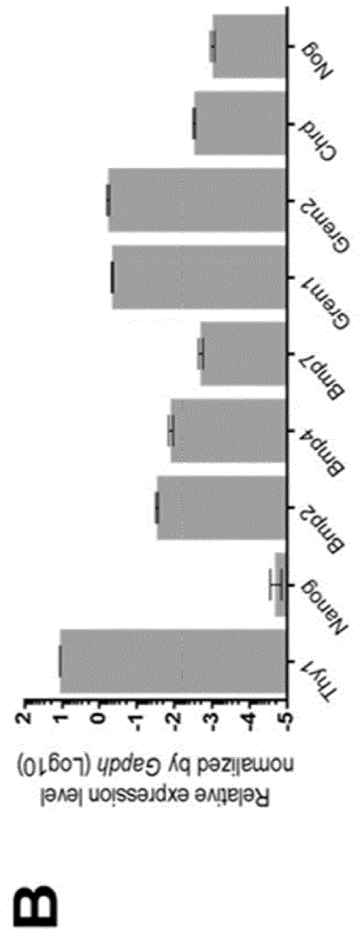

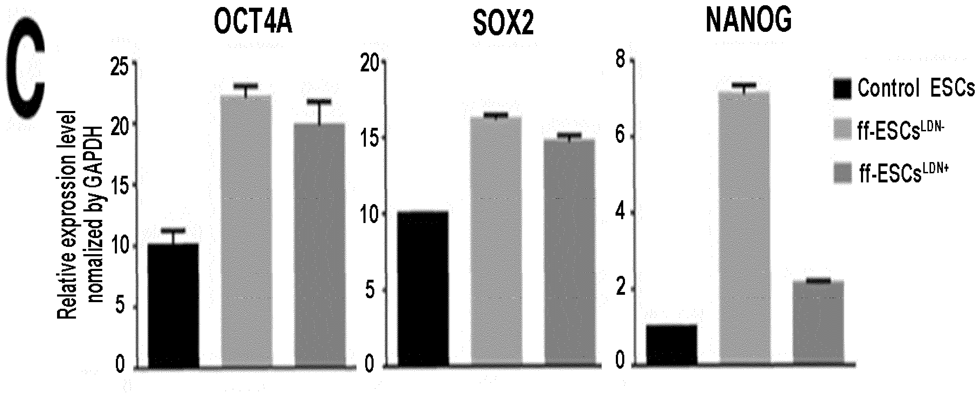

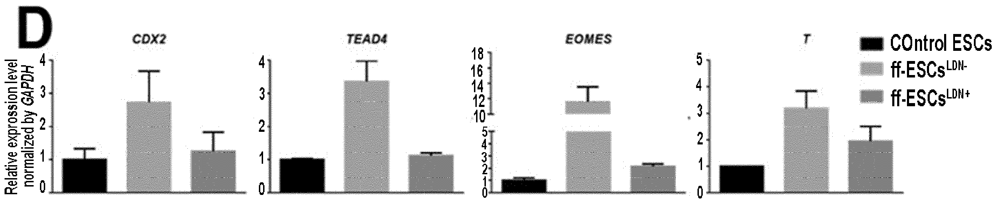

Figure 2 shows the results of analyzing various gene expressions in porcine stem cells cultured without feeder cells (ff-ESCs^{LDN-}, ff-ESCs^{LDN+}) and porcine embryonic stem cells cultured with feeder cells (control). Figure 2A is the results showing the immunological staining of pluripotency genes and the SMAD signaling system; Figure 2B is the result showing the expression level of SMAD signaling system-related genes secreted from feeder cells; Figure 2C shows the results of qPCR analysis for the expression of the pluripotency marker genes; and Figure 2D shows the results of qPCR analysis for the expression of the trophectoderm marker genes.
Figure 3 shows the results of analyzing the optimal concentration of LDN-193189 in porcine embryonic stem cells cultured without feeder cells. Figure 3A shows the results of annexin V staining treatment with subdivided concentration of LDN-193189; Figure 3B is the result showing the apoptosis rate due to the annexin V staining treatment; Figure 3C shows the result of WST-8 assay; and Figure 3D shows the result of Ki-67 staining.
Figure 4 shows the results of analyzing the expression of pluripotency-related genes by qPCR in LDN-193189-treated porcine embryonic stem cells cultured at various concentrations of FGF2, activin A and CHIR99021.

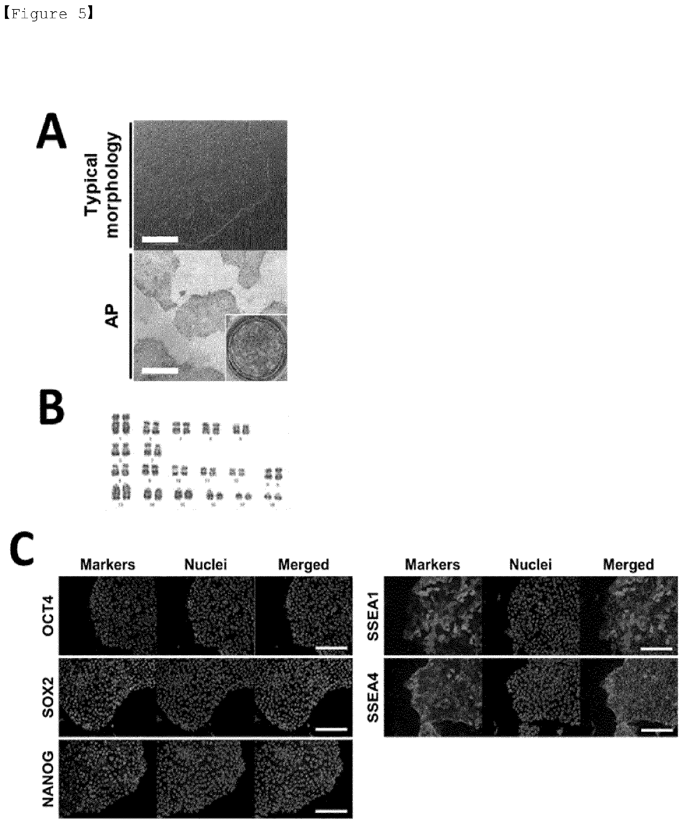

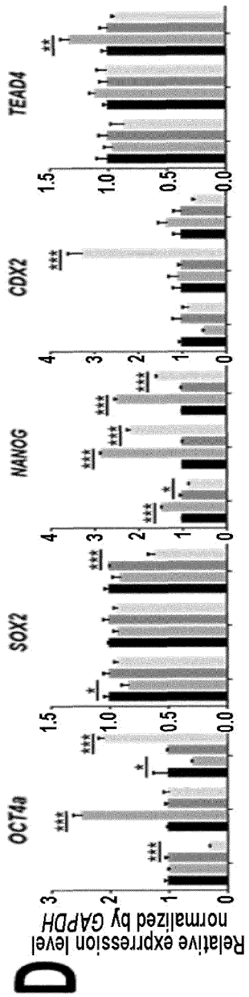

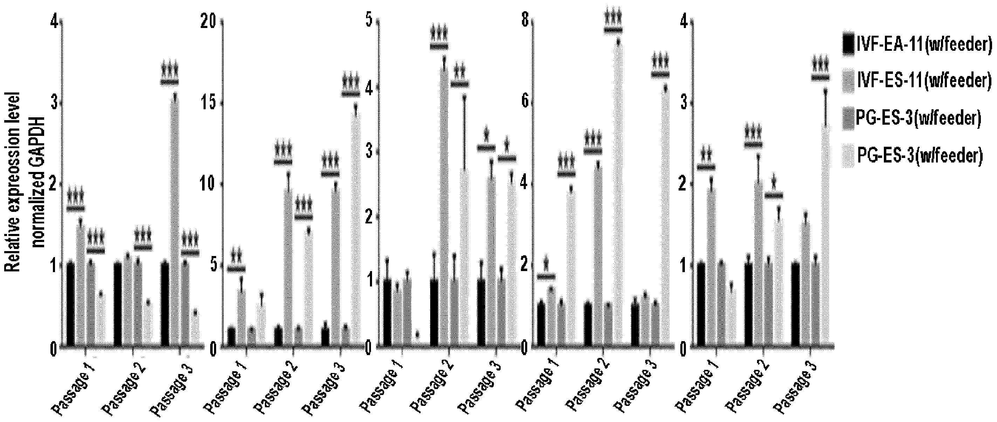

Figure 5 shows the results of analyzing the characteristics of porcine embryonic stem cells cultured without feeder cells. Figure 5A shows the results of typical morphology and AP staining (bottom, AP staining image; scale bar, 400 µm) of porcine embryonic stem cells; Figure 5B shows the karyotype of porcine embryonic stem cells; Figure 5C shows the results of immunostaining for pluripotency marker genes in porcine embryonic stem cells (scale bar, 200 µm); and Figure 5D shows the results of qPCR analysis of pluripotency and differentiation marker gene expression in porcine embryonic stem cells cultured without feeder cells.
Figure 6 relates to embryoid bodies derived from porcine embryonic stem cells cultured without feeder cells, wherein Figure 6A shows the morphology of embryoid bodies derived from porcine embryonic stem cells (scale bar, 400 µm), and Figure 6B shows the results of qPCR analysis of pluripotency and differentiation marker gene expression in porcine embryonic stem cells and differentiated cells.
Figure 7 shows the results of histological analysis of teratomas formed from porcine embryonic stem cells.
Figure 8 shows the results of culturing porcine embryonic stem cells in various extracellular matrices such as fibronectin, laminin, poly-L-lysine or type 1 collagen.
Figure 9 shows the results of analyzing the function of LDN-193189 in culture medium in which KSR was replaced with FBS.
Figure 10 is the results showing the effect of LDN-193189 on porcine embryonic stem cells grown on feeder cells.

### [Best Mode]

Hereinafter, the present invention will be described in more detail.

Unless defined otherwise, all technical terms used in the present invention have the same meaning as commonly understood by a person having ordinary knowledge in the relevant field of the present invention. In addition, preferred methods or samples are described in the present specification, but similar or equivalent ones are also included in the scope of the present invention. The contents of all publications described by reference in the present specification are incorporated herein by reference in their entirety.

In describing and claiming particular features of the present disclosure, the following terms will be used in accordance with the definitions set forth below unless otherwise specified.

It should be understood that although some aspects in the present specification are described with the term "comprising," other similar aspects described in terms of "consisting of" and/or "consisting essentially of" are also provided.

In the present invention, the term "medium," "culture medium," "medium for culture," "medium composition" or "composition for culture" refers to a culture medium containing nutrients capable of supporting the growth and survival of stem cells in *in vitro* culture conditions, and may be used interchangeably without distinction in the present specification.

In the present invention, "culturing porcine pluripotent stem cells" means that porcine pluripotent stem cells proliferate without differentiating into cells of a specific lineage, while maintaining pluripotency/stemness.

Porcine pluripotent stem cells may be porcine embryonic stem cells or porcine induced pluripoetent stem cells, and the pluripotent stem cells may generally be obtained using any method known in the art.

In the present invention, the term "embryonic stem cells" refers to cells cultured *in vitro* by extracting the inner cell mass from a blastula stage embryo just before the fertilized ovum implants in the mother's uterus, which may be pluripotent or totipotent, capable of differentiating into cells of all tissues of an individual, and in a broad sense also include embryoid bodies derived from embryonic stem cells. Embryoid bodies are intermediate structures formed by stem cells during the spontaneous differentiation of embryonic stem cells into various tissue types, and are in the form of aggregates formed during culture of embryonic stem cells. Meanwhile, the embryonic stem cells of the present invention are porcine embryonic stem cells derived from pigs.

Embryonic stem cells may differentiate into ectodermal, mesodermal and endodermal stem cells.

In the present invention, the term "differentiation" refers to a phenomenon in which the structure or function of a cell becomes specialized during cell division, proliferation and growth. Pluripotent stem cells may differentiate into lineage-limited progenitor cells (e.g., ectodermal cells, mesodermal cells, endodermal cells, and the like), then further differentiate into other types of progenitor cells (e.g., hemangioblasts, and the like), and then differentiate into terminally differentiated cells (e.g., vascular endothelial cells, vascular smooth muscle cells, and the like) that play characteristic roles in specific tissues (e.g., blood vessels, and the like).

Thus, the pluripotency of the porcine pluripotent embryonic stem cells of the present invention may be analyzed by whether they may differentiate into ectodermal, mesodermal and endodermal cells, when a certain number of porcine embryonic stem cells cultured using the medium composition of the present invention are transplanted into immunosuppressed mice.

In the present invention, the term "induced pluripotent stem cells" or "iPSCs" refers to cells that have acquired pluripotency by processing somatic cells or already differentiated cells. Wherein processing methods include, but are not limited to, compounds, genetic transformation, culturing methods under specific conditions, and the like. "Porcine induced pluripotent stem cells" or "piPSCs" refer to cells that have acquired pluripotency by processing porcine somatic cells or porcine differentiated cells.

In the present invention, the term "passage culture" refers to a method of continuously culturing cells from generation to generation by periodically transferring a portion of the cells to a new culture vessel and then changing a culture medium, in order to continuously culture the cells for a long period of time in a healthy state. The term "passage" refers to the growth into pluripotent stem cells from the initial seed culture in a culture vessel to the time when the cells actively grow in the same culture vessel (confluence). It is used as a method to increase the number of healthy cells because growth nutrients are consumed or contaminants build up and cells naturally die when a certain period of time elapses while the number of cells increases in a culture vessel with a limited space, and usually, replacing the medium (culture vessel) once or culturing by dividing into cell groups is called 1 passage. Methods of passage culture may use methods known in the art without limitation, but may be preferably performed by mechanical separation or enzymatic separation.

As a result of continuing research using various methods to develop a medium composition for culturing porcine pluripotent stem cells, the present inventors have developed a medium composition for culturing porcine pluripotent stem cells comprising LDN-193189.

In one embodiment of the present invention, the LDN-193189 is included at a concentration of 10 nM to 1000 nM.

In one embodiment of the present invention, the porcine pluripotent stem cells are porcine embryonic stem cells or porcine induced pluripotent stem cells.

In one embodiment of the present invention, the medium composition for culturing porcine pluripotent stem cells may be used for feeder cell-free culture of porcine pluripotent stem cells.

In one embodiment of the present invention, the feeder cell-free culture is culturing the cells in a culture vessel coated with extracellular matrix (ECM) or Matrigel^{®} or a replacement thereof.

In one embodiment of the present invention, the feeder cell-free culture is culturing the cells in a culture vessel coated with fibronectin, laminin, poly-L-lysine, type 1 collagen or Matrigel^{®}.

In one embodiment of the present invention, the medium composition for culturing porcine pluripotent stem cells may further comprise FGF2, activin A and CHIR99021.

In one embodiment of the present invention, the concentration of FGF2 may be 0.1 ng/ml to 100 ng/ml, the concentration of activin A may be 0.1 ng/ml to 10 ng/ml, the concentration of CHIR99021 may be 0.1 µM to 4.5 µM, and the concentration of IWR-1 may be 0.1 µM to 5 µM.

In one embodiment of the present invention, the medium composition for culturing porcine pluripotent stem cells promotes the proliferation of porcine pluripotent stem cells and inhibits apoptosis.

In one embodiment of the present invention, the medium composition for culturing porcine pluripotent stem cells increases the number of porcine pluripotent stem cell colonies and increases the size of the colonies.

In one embodiment of the present invention, the medium composition for culturing porcine pluripotent stem cells enhances stemness/pluripotency of porcine pluripotent stem cells.

In one embodiment of the present invention, the medium composition for culturing porcine pluripotent stem cells may comprise 0.1 to 3% by weight of serum-free or serum components.

In one embodiment of the present invention, a composition for addition to a porcine pluripotent stem cell culture medium comprising LDN-193189 as an active ingredient is provided.

In one embodiment of the present invention, the LDN-193189 may be included at a concentration of 10 nM to 1000 nM in the composition for addition to the porcine pluripotent stem cell culture medium.

The present invention provides a method of culturing porcine pluripotent stem cells, comprising treating porcine pluripotent stem cells with the medium composition for culturing porcine pluripotent stem cells according to the present invention and culturing them, wherein the porcine pluripotent stem cells are porcine embryonic stem cells or porcine induced pluripotent stem cells.

In one embodiment of the present invention, the method of culturing porcine pluripotent stem cells may be a feeder cell-free culture.

In one embodiment of the present invention, the feeder cell-free culture may be culturing the cells in a culture vessel coated with extracellular matrix (ECM) or Matrigel.

In one embodiment of the present invention, the feeder cell-free culture may culture the cells in a culture vessel coated with fibronectin, laminin, poly-L-lysine, type 1 collagen or Matrigel.

The extracellular matrix may include extracellular matrices commonly used in the art in addition to fibronectin, laminin, poly-L-lysine and type 1 collagen.

The medium composition for culturing porcine pluripotent stem cells according to the present invention comprises LDN-193189, thereby having the effects of promoting the proliferation or growth of porcine pluripotent stem cells, inhibiting stem cell apoptosis, increasing the number and size of stem cell colonies and enhancing the stemness and differentiation potential of stem cells.

In the medium composition for culturing porcine pluripotent stem cells according to the present invention, the LDN-193189 may be used by adding it to the medium used for culturing stem cells.

LDN-193189 is a bone morphogenetic (BMP) pathway inhibitor, and is known to inhibit ALK1, ALK2, ALK3 and ALK6. LDN-193189 is known to induce differentiation of human pluripotent stem cells into neural progenitor cells or pancreatic cells, and is known to induce differentiation of mouse pluripotent stem cells.

However, in the present invention, it was discovered that LDN-193189 has the effects of maintaining stemness/pluripotency and inhibiting differentiation of porcine pluripotent stem cells. These effects are contrary to the existing effects of LDN-193189, and indicate that the mechanism for maintaining stemness is different depending on the species of embryonic stem cells, and that the medium for culturing embryonic stem cells should also be used differently depending on the species of embryonic stem cells.

The concentration of LDN-193189 included in the medium composition for culturing porcine pluripotent embryonic stem cells is 10 nM to 1000 nM. If the concentration of LDN-193189 is less than 10 nM, there is no particular effect, and if it is more than 1000 nM, it shows cytotoxicity. LDN-193189 included in the medium composition for culturing porcine pluripotent embryonic stem cells according to one embodiment of the present invention has, but is not limited to, a concentration of 100 nM.

The medium composition for culturing porcine pluripotent stem cells according to the present invention may further comprise FGF2, activin A, CHIR99021 and IWR-1.

FGF2, activin A, CHIR99021 and IWR-1 further included in the medium composition for culturing porcine pluripotent stem cells according to one embodiment of the present invention have concentrations of 0.1 ng/ml to 100 ng/ml, 0.1 ng/ml to 10 ng/ml, 0.1 µM to 4.5 µM and 0.1 µM to 5 µM, respectively. If the concentration of FGF2 is less than 0.1 ng/ml, there is no particular effect, and if it is more than 100 ng/ml, there is no additional effect due to increased concentration or it is toxic to cells. If the concentration of activin A is less than 0.1 ng/ml, there is no particular effect, and if it is more than 10 ng/ml, there is no additional effect due to increased concentration or it is toxic to cells. If the concentration of CHIR99021 is less than 0.1 µM, there is no particular effect, and if it is more than 4.5 µM, it is toxic to cells. If the concentration of IWR-1 is less than 0.1 µM, there is no particular effect, and if it is more than 5 µM, there is no additional effect due to increased concentration or it is toxic to cells. FGF2, activin A, CHIR99021 and IWR-1 included in the medium composition for culturing porcine pluripotent stem cells according to one embodiment of the present invention have, but are not limited to, concentrations of 20 ng/ml, 5 ng/ml, 0.5 µM and 0.5 µM, respectively.

The basal medium for culturing porcine pluripotent stem cells according to the present invention may be used without limitation as long as it is a medium widely known to those skilled in the art. The basal medium may be prepared by artificial synthesis, or a commercially prepared medium may be used. Examples of commercially prepared media include, but are not limited to, Dulbecco's modified Eagle's medium (DMEM), minimal essential medium (MEM), basal medium eagle (BME), RPMI 1640, F-10, F-12, α-minimal essential medium (α-MEM), Glasgow's minimal essential medium (G-MEM), Isocove's modified Dulbecco's medium (IMDM) and DMEM/F-12.

In one embodiment of the present invention, DMEM/F-12 basal medium is used.

In the medium composition for culture according to the present invention, it is preferable to additionally use antibiotics and/or antimycotics to prevent infection by bacteria, fungi, and the like, and/or substances commonly used in the art to prevent the growth of mycoplasma. As the antibiotics, all antibiotics commonly used in cell culture, such as penicillin-streptomycin, may be used; as the antimycotics, commonly used substances, such as amphotericin B, may be used; as the mycoplasma inhibitor, commonly used substances, such as gentamicin, ciprofloxacin and azithromycin, may be used, but they are not limited thereto. In addition, a commercially available antibiotic-antimycotic (AA) (Gibco) may be used.

In addition, the composition for culture according to the present invention may comprise 1% glutamax (or glutamine) and 0.1 mM beta-mercaptoethanol.

The medium composition for culturing porcine pluripotent stem cells according to the present invention may be used for feeder cell-free culture of porcine pluripotent stem cells.

In general, feeder cells such as mouse fibroblasts have been used to maintain pluripotency or stemness when culturing pluripotent stem cells or embryonic stem cells (feeder cell-dependent culture), but due to problems such as *in vitro* contamination/cell-to-cell variation, attempts have been made to perform feeder cell-free mass culture of pluripotent stem cells. However, in the case of porcine pluripotent stem cells, pluripotency/stemness was not maintained when feeder cell-free culture medium used for human embryonic stem cells was used. However, it was confirmed that the pluripotency/stemness of porcine pluripotent stem cells was maintained even without feeder cells when the medium composition for culturing porcine pluripotent stem cells according to the present invention was used.

In addition, the medium composition for culturing porcine pluripotent stem cells according to the present invention may comprise 0.1 to 3% by weight of serum-free or serum components.

The serum-free medium refers to any culture medium that does not contain a certain amount or more of serum derived from animals, including humans (animal-derived serum). For example, the serum-free medium may contain less than 0.1% by weight or less than 0.01% by weight of animal-derived serum based on the total composition content, and may specifically not contain animal-derived serum.

The present invention provides a composition for addition to a porcine pluripotent stem cell culture medium comprising LDN-193189 as an active ingredient.

By using the composition of the present invention, porcine pluripotent stem cells may be stably proliferated and cultured, and reproducible testing and production processes may be established.

In addition, the present invention relates to a method of culturing porcine pluripotent stem cells, comprising treating porcine pluripotent stem cells with the medium composition for culturing porcine pluripotent stem cells according to the present invention and culturing them.

The culture of porcine pluripotent stem cells may be a feeder cell-free culture, wherein the porcine pluripotent stem cells may be cultured in a culture vessel coated with, but not limited to, extracellular matrix or Matrigel^{®} or a replacement thereof (gelatin, collagen, laminin, fibronectin, poly-D-lysine, poly-L-lysine, or the like).

Unless otherwise indicated, all numbers used in the present specification and claims, whether recited or not, are to be understood as being modifiable by the term "about" in all instances. It is also to be understood that the precise numbers used in the specification and claims form additional embodiments of the present disclosure. Efforts have been made to ensure the accuracy of the numerical values disclosed in the examples. However, all measured values may inherently include certain error values generated from the standard deviations measured in their respective measurement techniques.

### [Best Mode]

Hereinafter, the present invention will be described in more detail through examples. It will be apparent to those skilled in the art that these examples are only for illustrating the present invention in more detail and the scope of the present invention is not limited to these examples in accordance with the gist of the present invention.

### Example 1. Analysis of culturability of porcine embryonic stem cells according to culture medium

### Example 1-1. Isolation of porcine embryonic stem cells

The management and experimental use of animals were approved by the Institutional Animal Care and Use Committee (IACUC) at Seoul National University (Approval Nos: SNU-191025-4-4 and SNU-201019-1-2). Pregnant ICR mice and athymic nude mice were purchased from Samtaco Bio Inc. (Korea) and OrientBio Inc. (Korea), respectively. Mice were maintained according to the standard protocols of the Institute of Laboratory Animal Resources at Seoul National University.

Isolation of porcine embryonic stem cells from blastocysts and production of feeder cells using mouse fibroblasts were performed by the method of KR10-2142400.

### Example 1-2. Feeder cell-dependent culture of porcine embryonic stem cells

The "embryonic stem cell culture medium" used to culture porcine embryonic stem cells with feeder cells is DMEM/F12 basal medium containing 15% (v/v) knock-out serum replacement (KSR), 0.1% (v/v) chemically defined lipid concentrate (LC), 1X GlutaMAX^{®}, 0.1 mM β-mercaptoethanol, 1X MEM non-essential amino acids, and 1X antibiotics-antimycotics (all of the aforementioned materials were purchased from Gibco, Gaithersburg, MD, USA), 20 ng/mL hrFGF2 (fibroblast growth factor 2) (R&D Systems, Minneapolis, MN, USA), 5 ng/mL ActA (activin; activin A) (R&D Systems), 1.5 µM CHIR99021 (CH, Cayman Chemical, Ann Arbor, MI, USA) and 2.5 µM IWR-1 (Sigma-Aldrich, St. Louis, MO, USA) .

Porcine embryonic stem cells were passage cultured with feeder cells every 5-7 days. 24 hours before passage culture, porcine embryonic stem cells were cultured in the previously mentioned "embryonic stem cell culture medium" containing 10 µM Y-27632 (Santa Cruz Biotechnology, Dallas, TX, USA). Colonies of sufficiently grown embryonic stem cells were isolated into small clumps using TrypLE^{®} Express (Gibco). These cell clumps are transferred to new feeder cells, and cultured for 24 hours in "embryonic stem cell culture medium" containing 10 µM Y-27632 (Santa Cruz Biotechnology). After 24 hours, the embryonic stem cells adhered to the feeder cells are cultured in "embryonic stem cell culture medium" without Y-27632 for 4-6 days. The medium was changed every 24 hours and cultured under conditions of 5% CO₂ and 37°C.

### Example 1-3. feeder cell-free culture of porcine embryonic stem cells

For feeder-free culture of porcine embryonic stem cells, porcine embryonic stem cells, which had been cultured on feeder cells, were cultured on a Matrix^{™}-coated culture vessel (SPL Life Sciences, Pocheon, Korea) at a split ratio of 1:5 to 1:10 using embryonic stem cell culture medium for feeder cell-free culture (hereinafter referred to as "FF-embryonic stem cell culture medium").

FF-embryonic stem cell culture medium is DMEM/F12 basal medium containing 15% (v/v) KSR, 0.1% (v/v) LC, 1X Glutamax, 0.1 mM β-mercaptoethanol, 1X MEM non-essential amino acids, and 20 ng/mL hrFGF2 (R&D Systems), 5 ng/mL ActA (R&D Systems), 1.5 µM CHIR99021 (CH, Cayman Chemical), 2.5 µM IWR-1 (Sigma-Aldrich), 1X antibiotics-antimycotics (all of the aforementioned materials were purchased from Gibco, Gaithersburg, MD, USA) and 100 nM LDN-193189 (Cayman Chemical).

Porcine embryonic stem cells were passage cultured every 5-7 days. 24 hours before passage culture, porcine embryonic stem cells were cultured in "FF-embryonic stem cell culture medium" containing 10 µM Y-27632 (Santa Cruz Biotechnology, Dallas, TX, USA). Next, the cultured stem cells were isolated into small clumps using TrypLE^{®} Express (Gibco). These cells were transferred to a new Matrix^{™}-coated plate, and cultured for 24 hours in "FF-embryonic stem cell culture medium" containing 10 µM Y-27632 (Santa Cruz Biotechnology). Next, the adherent stem cells were cultured in "FF-embryonic stem cell culture medium" without Y-27632 for 4-6 days. The medium was changed every 24 hours and cultured under conditions of 5% CO₂ and 37°C.

### Example 1-4. Analysis of the effect of LDN-193189 on feeder cell-free culture of porcine embryonic stem cells (cell morphology analysis)

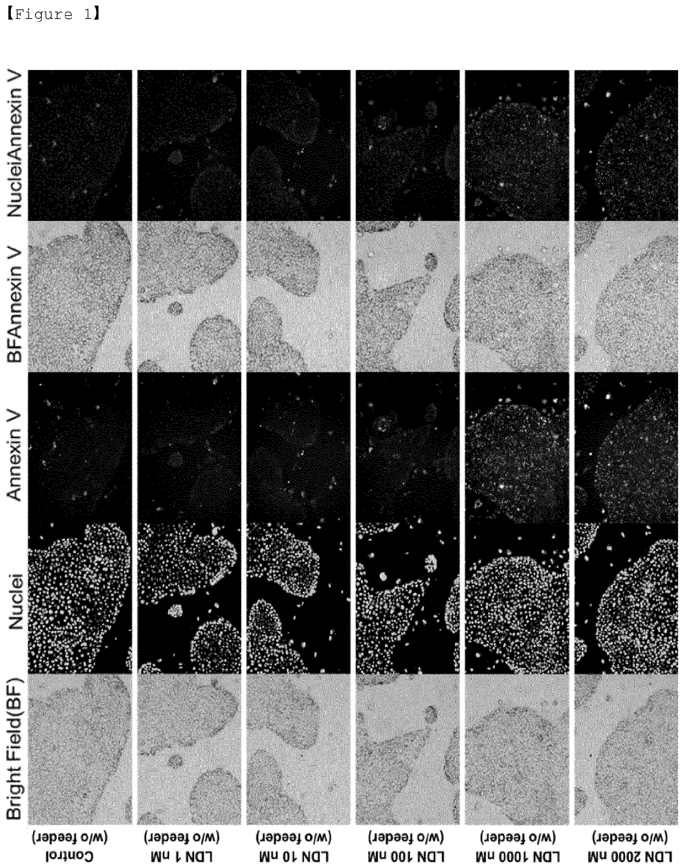

To analyze the effect of LDN-193189, which is an ALK2/3 inhibitor, on feeder cell-free culture of porcine embryonic stem cells, LDN-193189 cytotoxicity was confirmed through annexin V staining (Figure 1). To find the optimal concentration of LDN-193189 treatment, a concentration-dependent cytotoxicity experiment was conducted, and annexin V staining showed that LDN-193189 had high cytotoxicity at a concentration of 1000 nM or more. In addition, treatment at a concentration of 1000 nM or more for 24 hours or more induced apoptosis of most cells.

### Examples 1-5. Analysis of the effect of LDN-193189 treatment on feeder cell-free culture of porcine embryonic stem cells (gene expression analysis)

To analyze the expression level of pluripotency-related genes, differentiation-related genes, and the like depending on the culture period, porcine embryonic stem cells were cultured using "FF-embryonic stem cell culture medium" containing 100 nM LDN-193189 (ff-ESCs^{LDN+}) and "FF-embryonic stem cell culture medium" not containing LDN-193189 (ff-ESCs^{LDN-}) , without feeder cells, and porcine embryonic stem cells were cultured as a control using the aforementioned embryonic stem cell culture medium along with feeder cells (control ESCs).

The cells were obtained on days 2, 4 and 6 after plating using each medium. Total RNA was extracted using TRIzol reagent (Invitrogen, Carlsbad, USA) and cDNA was synthesized using High-Capacity RNA-to-cDNA Kit (Applied Biosystems, Foster City, USA) . cDNA was amplified using the DyNAmo HS SYBR Green qPCR kit (Thermo Fisher Scientific, Waltham, USA) with the primer sets listed in Table 1.

**[Table 1]**

| **Species** | **Genes** | **Sequences** | **Annealing Tm (°C)** | **Size (bp)** |
|---|---|---|---|---|
| | ***OCT4a*** | 5'- CTTGGAGAGCCCTGGTTTTACT -3' | 64 | 159 |
| | | 5'- GCCAGGTCCGAGGATCAAC -3' | | |
| | ***SOX2*** | 5'- CGGCGGTGGCAACTCTAC -3' | 64 | 100 |
| | | 5'- TCGGGACCACACCATGAAAG -3' | | |
| | ***NANOG*** | 5'- CATCTGCTGAGACCCTCGAC -3' | 60 | 195 |
| | | 5'- GGGTCTGCGAGAACACAGTT -3' | | |
| | ***CDX2*** | 5'- GCCAAGTGAAAACCAGGACGA -3' | 60 | 120 |
| | | 5'- GCTCGGCCTTTCTCCGAATG -3' | | |
| | ***TEAD4*** | 5'- GTCGGCGCAAGATCATCCTA -3' | 60 | 159 |
| | | 5'- CCTGGATCTCACGGGCTTTT -3' | | |
| | ***EOMES*** | 5'- CCAACCACGTCTGCACATTG -3' | 60 | 206 |
| | | 5'- AAGCGGTGTACATGGAGTCG -3' | | |
| **Pig** | ***T*** | 5'- CTGTGAGAGGTATCCTGCCCT -3' | 64 | 184 |
| | | 5'- GGGACTCATGGGAAACATGC -3' | | |
| | **BMP2** | 5'- GAAGGAAGGGAACCCGGTC -3' | 60 | 84 |
| | | 5'- CGGGGACACGTCCATTGAAA -3' | | |
| | **BMP4** | 5'- CGTTGGTCTCGAGTATCCCG -3' | 60 | 106 |
| | | 5'- AGAGTTTTCGCTGGTCCCTG -3' | | |
| | ***BMP* 7** | 5'- ACTTCGACGACAGCTCCAAC -3' | 60 | 200 |
| | | 5'- AAATACCCTCACACGCCTGC -3' | | |
| | ***PAX6*** | 5'- AGAGAAGACAGGCCAGCAAC -3' | 60 | 169 |
| | | 5'- GGCAGAGCACTGTAGGTGTT -3' | | |
| | ***AMY2*** | 5'- TGCTCTTGAATGTGAGCGGT -3' | 60 | 206 |
| | | 5'- TACGGACGCCAACGTTGTTA -3' | | |
| | ***GAPDH*** | 5'- TGCTCCTCCCCGTTCGAC -3' | 60 | 100 |
| | | 5'- ATGCGGCCAAATCCGTTC -3' | | |

As shown in Figure 2, changes in the characteristics of porcine embryonic stem cells were confirmed during feeder cell-free culture. Figure 2A is the results showing the immunological staining of pluripotency genes and the SMAD signaling system, and Figure 2B is the result showing the expression level of SMAD signaling system-related genes secreted from feeder cells.

In the culture of porcine embryonic stem cells, if essential feeder cells are removed, stemness is lost, and differentiation into other cells is induced. In the case of early embryos, they are programmed to differentiate into mesodermal tissues, and the expression of SMAD signaling protein, which is one of the signaling systems involved in this, was confirmed. In addition to the phosphor-SMAD2/3 proteins involved in stem cell proliferation, the expression of phosphor-SMAD1/5 proteins involved in differentiation was confirmed. In fact, it was confirmed that various genes (Grem1, Grem2, Chrd, Nog) that suppress the activity of this signaling system were expressed in feeder cells involved in maintaining pluripotency of embryonic stem cells (Figures 2A and 2B).

In addition, as shown in C and D of Figure 2, the effect of treatment with LDN-193189 (250 nM), which is an inhibitor of the SMAD1/5 signaling system, on feeder cell-free culture of porcine embryonic stem cells was confirmed.

To prove the hypothesis that the inhibitory gene of the SMAD1/5 signaling system secreted from feeder cells helps maintain pluripotency of porcine embryonic stem cells, porcine embryonic stem cells cultured without feeder cells were treated with LDN-193189, which is an inhibitor of the SMAD1/5 signaling system. As a result, it was confirmed that genes involved in pluripotency and mesodermal tissue development, which had changed when the feeder cells were removed, were restored to the level when cultured on the feeder cells.

### Example 2. Analysis of the optimal concentration of LDN-193189 and the effect of FGF2, activin 2 and CHIR99021 depending on concentration in feeder cell-free culture of porcine embryonic stem cells

### Example 2-1. Analysis of optimal concentration of LDN-193189

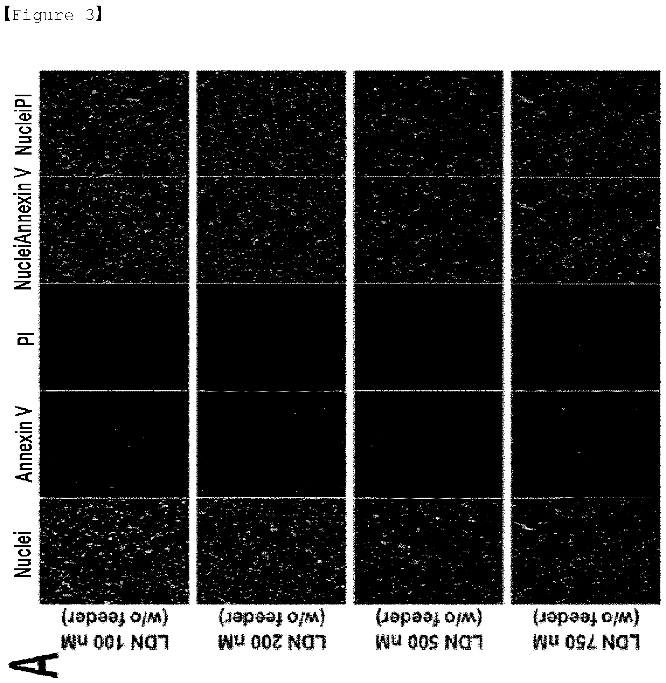

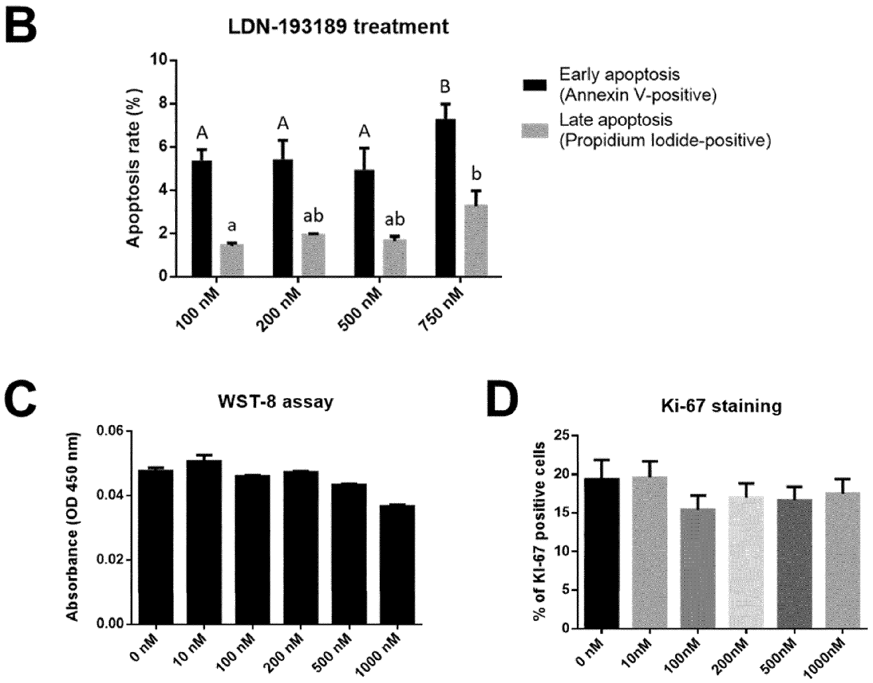

To confirm the optimal concentration of "FF-embryonic stem cell culture medium" containing LDN-193189 from 100 nM to 1000 nM, the concentration was subdivided and annexin V staining was performed (Figure 3A). It was confirmed that the apotosis rate significantly increased at concentrations of 750 nM or more (Figure 3B). Ki-67 staining results showed no significant difference from 100 nM to 1000 nM (Figure 3D), but WST-8 results confirmed a significant decrease in cell activity at concentrations of 500 nM or more (Figure 3C). In fact, LDN-193189 is known to inhibit the activin A signaling system involved in stem cell proliferation at high concentrations. Therefore, the final treatment concentration was determined to be 100 nM, which is the maximum concentration that inhibits only the BMP signaling system and does not cause cytotoxicity.

### Example 2-2. Analysis of FGF2, activin 2 and CHIR99021 depending on concentration

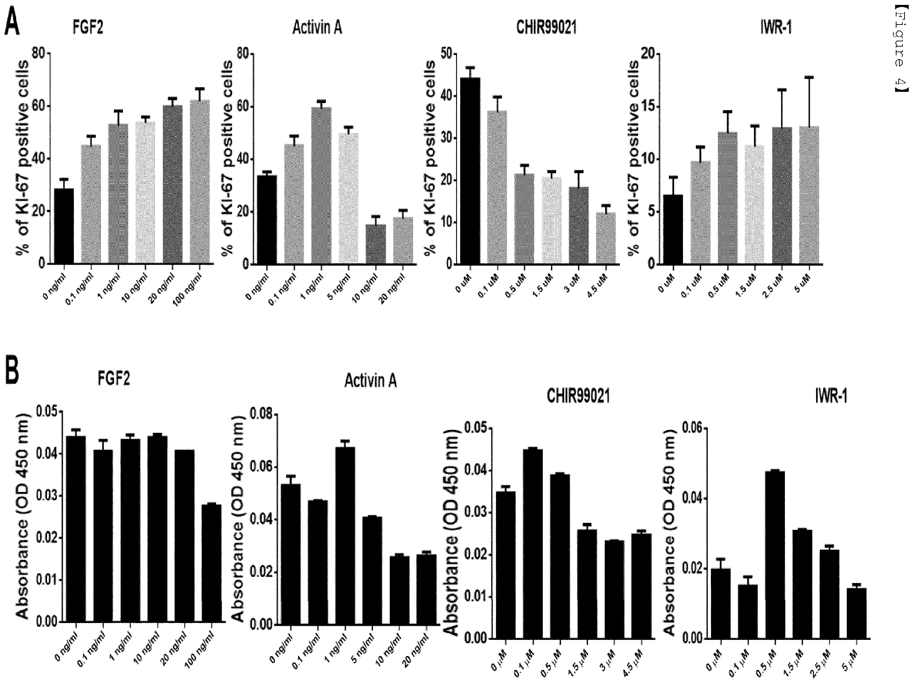

To optimize the concentration of signaling molecules contained in the culture medium, FGF2, activin A, CHIR99021 and IWR-1 substances were treated at various concentrations (Figures 4A and 4B). Figure 4A shows the results of confirming the proliferation rate through Ki-67 staining, and Figure 4B shows the results of confirming cell activity through WST-8 assay.

As a result of checking the proliferation rate through Ki-67 staining, the proportion of cells undergoing cell division gradually increased as the concentration of FGF2 increased. However, as a result of simultaneously measuring cell proliferation and cytotoxicity through the WST-8 assay, it was confirmed that cell activity decreased due to cytotoxicity at concentrations of 100 ng/ml or more.

It was confirmed that activin A showed decreased cytotoxicity and proliferation rate at concentrations of 10 ng/ml or more in both Ki-67 staining and WST-8 assay results. Therefore, it was confirmed that the optimal concentration of activin A was 1 ng/ml to 5 ng/ml.

The proliferation rate of CHIR99021 gradually decreased as the concentration increased, compared to the group not treated with CHIR99021. However, the results of WST-8 confirmed that the activity and proliferation rate of stem cells were improved when treated with the corresponding substances at a concentration of 0.1 µM to 0.5 µM.

As the concentration of IWR-1 increased, the proportion of cells undergoing cell division gradually increased. However, IWR-1 showed cytotoxicity at high concentrations, and showed the highest cellular activity when treated at a concentration of 0.5 µM.

### Example 3. Characterization of porcine embryonic stem cells cultured without feeder cells

For immunocytochemical analysis of porcine embryonic stem cells cultured without feeder cells, cell samples were left at 4°C for 10 min and fixed in 4% (w/v) paraformaldehyde for 30 min. After washing twice with Dulbecco's phosphate buffered saline (DPBS), the samples were treated with DPBS containing 10% (v/v) goat serum for 1 hour to prevent non-specific binding. Serum-treated cells were subject to immunocytochemical analysis for OCT4 (1:200; SC-9081, Santa Cruz Biotechnology), SOX2 (1:200; AB5603, Millipore, Billerica, MA, USA), NANOG (1:200; 500-P236, Peprotech, NJ, USA), SSEA1 (1:200; MAB4301, Millipore) and SSEA4 (1:200; MAB4304, Millipore). In case of nuclear proteins (e.g., OCT4, SOX2 and NANOG), fixed cells before applying antibodies were treated with 0.1% (v/v) Triton X-100 (Sigma-Aldrich) for 15 min before serum treatment. After treatment with the primary antibody, the cells were treated with Alexa Fluor-conjugated secondary antibody at room temperature for 3 hours. Nuclei of cells were identified with Hoechst 33342 (Molecular Probes, Eugene, OR, USA). The experimental results were visualized as images of stained cells using an inverted fluorescence microscope (Eclipse TE2000-U, Nikon, Konan, Japan).

For alkaline phosphatase (AP) staining, the cells were fixed in 4% (w/v) paraformaldehyde for 30 min and stained with a buffer solution containing nitro blue tetrazolium chloride and 5-bromo-4-chloro-3-indolyl phosphate toluidine salt stock solution (Roche, Basel, Switzerland) at room temperature for 30 min. Stained cells were visualized under an inverted microscope.

The appearance of the colonies of porcine embryonic stem cells cultured by treating "embryonic stem cell culture medium" containing LDN-193189 in a Matrigel^{®}-coated culture vessel without feeder cells was a flat monolayer, similar to the colonies of porcine embryonic stem cells cultured with feeder cells, and showed an epithelial cell morphology with a high nuclear-to-cytoplasm ratio, i.e., a typical embryonic stem cell morphology (Figure 5A). In addition, stem cells cultured without feeder cells had a normal karyotype (36+XX) (Figure 5B) and were stably maintained during long-term culture (10 passages or more).

Stem cells cultured without feeder cells showed alkaline phosphatase activity (bottom of Figure 5A) and expressed pluripotency markers (proteins) such as OCT4, SOX2, NANOG, SSEA1 and SSEA4, similar to porcine embryonic stem cells cultured with feeder cells (Figure 5C). qPCR analysis showed that during long-term culture of porcine embryonic stem cells without feeder cells, pluripotency-related genes such as OCT4, SOX2 and NANOG and trophoblast differentiation-related genes were stably expressed compared with genes in control porcine embryonic stem cells (Figure 5D).

(IVF-ES-11, PG-ES-3: porcine embryonic stem cell line; W/ feeder: group cultured on feeder cells; W/O feeder: group cultured without feeder cells)

### Example 4. Analysis of differentiation potential of porcine embryonic stem cells according to culture medium

### Example 4-1. Induction of differentiation of porcine embryonic stem cells using the embryoid body method

Embryonic stem cells cultured without feeder cells were isolated into single cells using TrypLE Express (Gibco) and suspension-cultured using DMEM containing 15% (v/v) FBS and 10 µM Y-27632 (treated only on day 1) without other cytokines for 5 days. After suspension culture, the dissociated cells aggregated to form embryoid bodies, which were adhesion-cultured for 2-3 weeks in DMEM containing 15% (v/v) FBS in a culture vessel coated with 0.1% (w/v) gelatin.

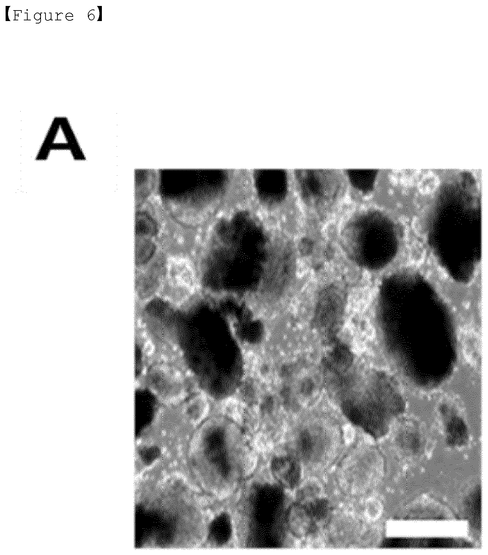

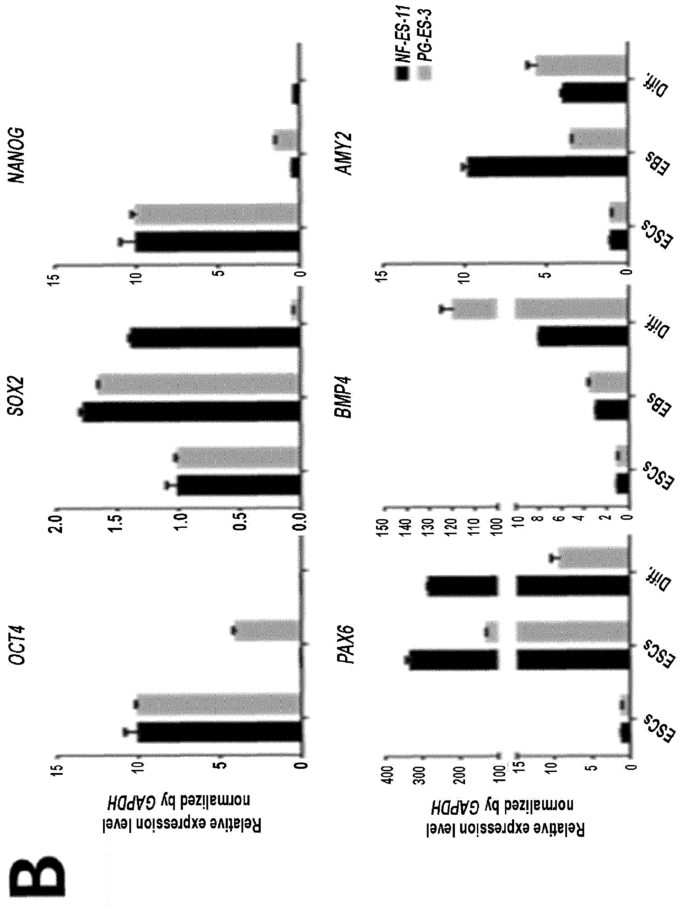

Porcine embryonic stem cells cultured without feeder cells formed embryoid bodies through suspension culture, which then adhered to the culture vessel and differentiated spontaneously (Figure 6A). Through *in vitro* differentiation, porcine embryonic stem cells highly expressed three germ layer markers (PAX6 [ectoderm], AMY2 [endoderm] and BMP4 [mesoderm]), but pluripotency marker genes were gradually downregulated as analyzed by qPCR (Figure 6B).

(IVF-ES-11, PG-ES-3: porcine embryonic stem cell line; ESCs: embryonic stem cells; Ebs: embryoid bodies; Diff: embryoid bodies differentiated *in vitro* by adhesion to a culture vessel)

### Example 5. Analysis of teratoma formation

Approximately 5-10 X 10⁶ porcine embryonic stem cells cultured without feeder cells were resuspended in 200 µL of "embryonic stem cell culture medium" containing 50% (v/v) BD Matrigel Matrix (BD Biosciences, Franklin Lakes, NJ, USA) and 10 µM Y-27632. Next, the resuspended porcine stem cells were subcutaneously injected into 5-week-old athymic nude mice (OrientBio). 2-3 months after transplantation, 1-2 cm teratomas were collected, fixed in 4% (w/v) paraformaldehyde, embedded in paraffin, and then stained with hematoxylin and eosin for light microscopy.

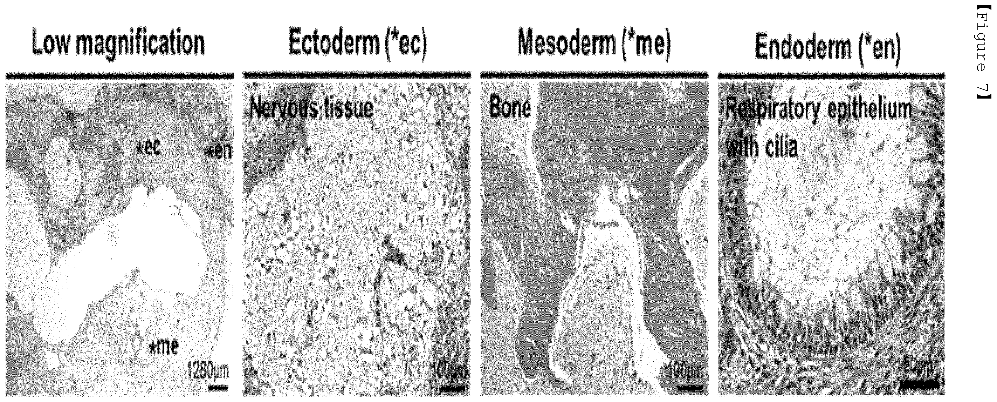

As a result, it was repeatedly confirmed that porcine embryonic stem cells cultured without feeder cells differentiated into teratomas when transplanted subcutaneously into immunodeficient mice (nude mice). The teratomas were histologically composed of various tissues representing three germ layers (Figure 7). Therefore, it was confirmed that porcine embryonic stem cells cultured without feeder cells also have pluripotent differentiation potential (ectoderm, mesoderm, endoderm).

### Example 6. Analysis of culture media in various extracellular matrices

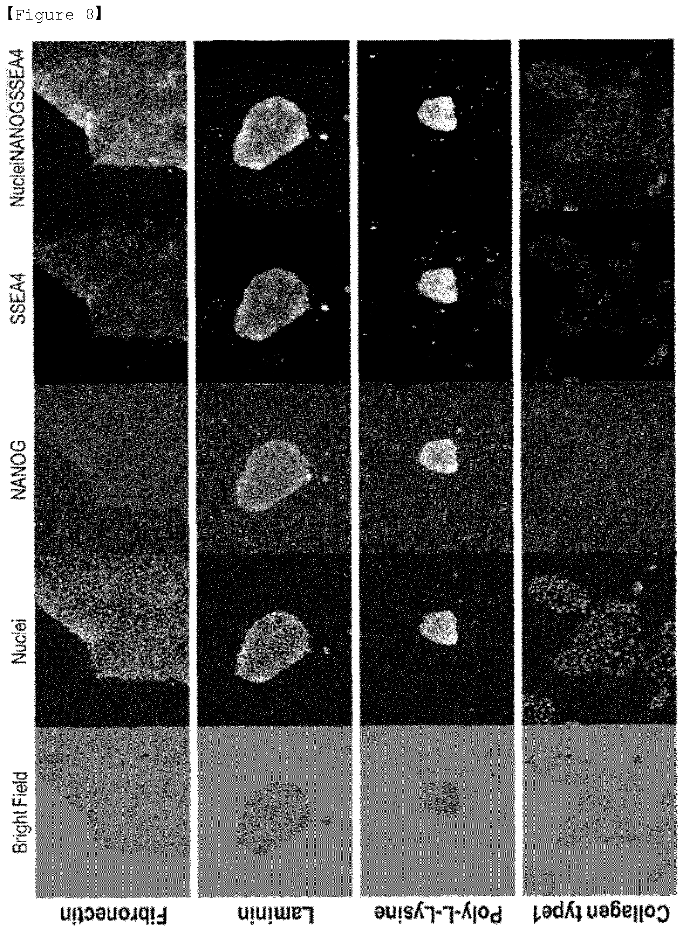

To evaluate the applicability of culture media for feeder cell-free culture of porcine embryonic stem cells in various extracellular matrices (ECMs), culture of stem cells was attempted in a cell culture vessel coated with fibronectin, laminin, poly-L-lysine or type 1 collagen. Figure 8 shows the results of culturing porcine embryonic stem cells in various extracellular matrices such as fibronectin, laminin, poly-L-lysine or type 1 collagen.

As a result of confirming the pluripotency markers NANOG and SSEA4 through immunological staining, it was confirmed that there were variations in characteristics such as pluripotency and proliferation potential of cells depending on the extracellular matrix, but the characteristics of porcine embryonic stem cells could be maintained on various substrates using a new culture medium.

### Example 7. Analysis of culture conditions comprising fetal bovine serum (FBS)

It was confirmed that treatment with LDN-193189 in medium containing KSR, which is a serum replacement, was effective in feeder cell-free culture of porcine embryonic stem cells.

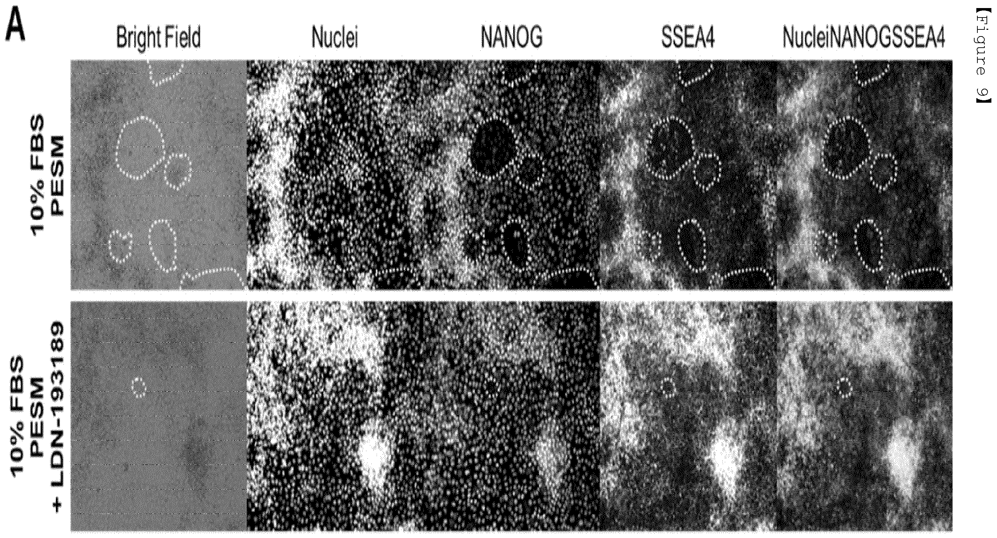

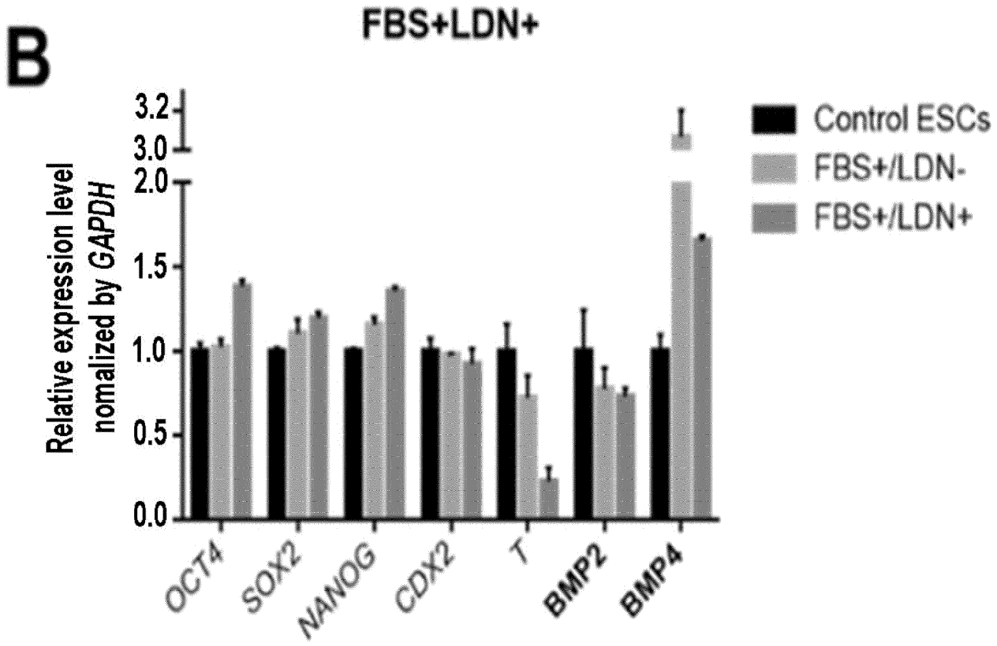

In addition, it was confirmed whether the substance helps maintain the pluripotency of porcine embryonic stem cells in a culture medium containing fetal bovine serum (FBS), rather than a serum replacement. Figure 9 shows the results of analyzing the function of LDN-193189 in a culture medium in which KSR was replaced with FBS, wherein Figure 9A shows the results of analyzing the characteristics of porcine embryonic stem cells through the pluripotency markers NANOG and SSEA4 (white dotted border: differentiated portion), and Figure 9B is a result showing changes in expression of genes related to pluripotency and mesodermal tissue development in culture medium containing FBS depending on whether LDN-193189 was treated.

As a result of confirming the pluripotency markers NANOG and SSEA4 through immunological staining, the culture medium containing FBS induced differentiation of porcine embryonic stem cells, and many cell colonies with reduced expression of NANOG and SSEA4 were discovered. This phenomenon was confirmed to be alleviated through treatment with LDN-193189, and qPCR analysis showed that treatment with LDN-193189 in an environment containing FBS reduced the expression of mesoderm-related genes, helping to maintain pluripotency of stem cells.

### Example 8. Confirmation of applicability in culture conditions with feeder cells

The effect of medium for feeder cell-free culture containing LDN-193189 on porcine embryonic stem cells grown on feeder cells was confirmed.

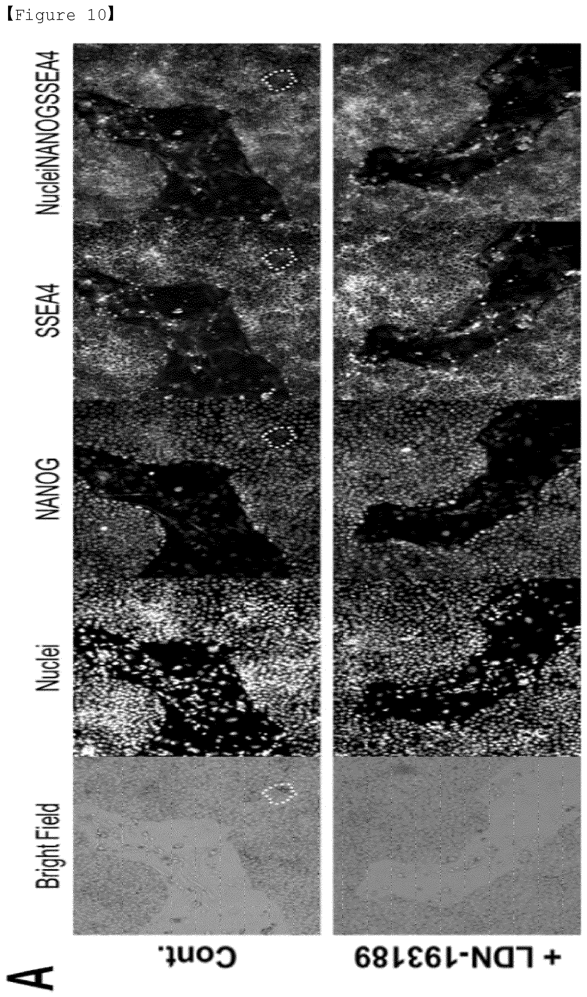

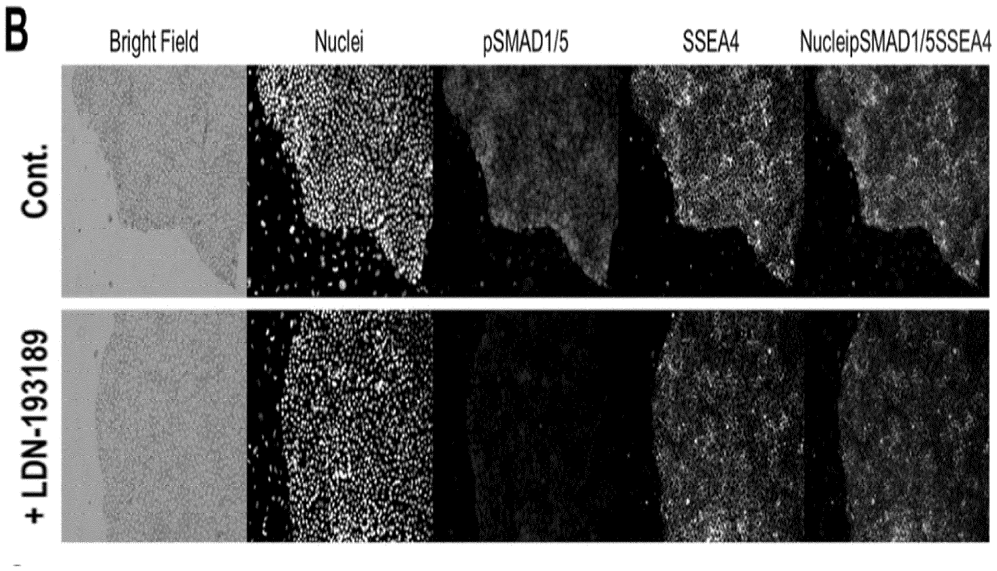

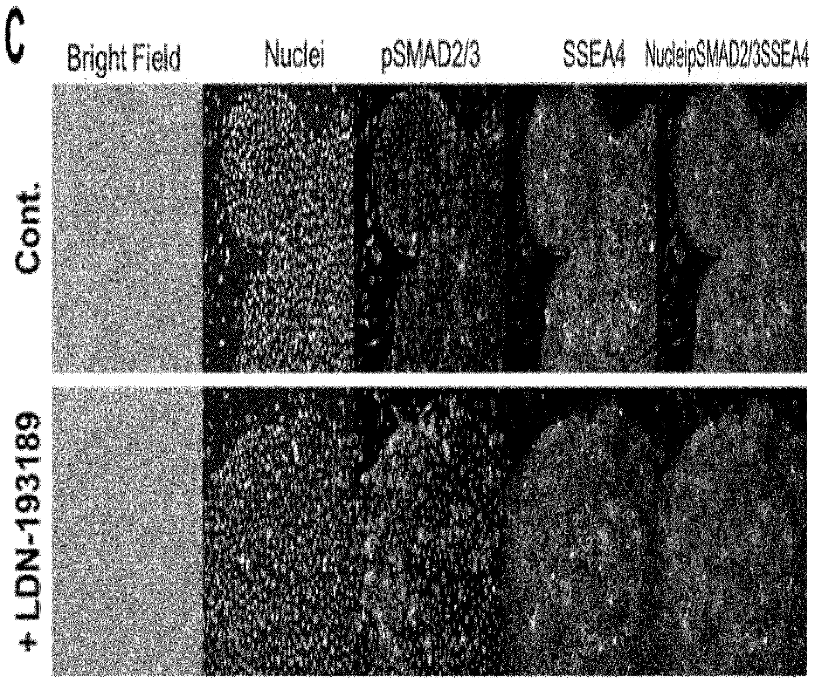

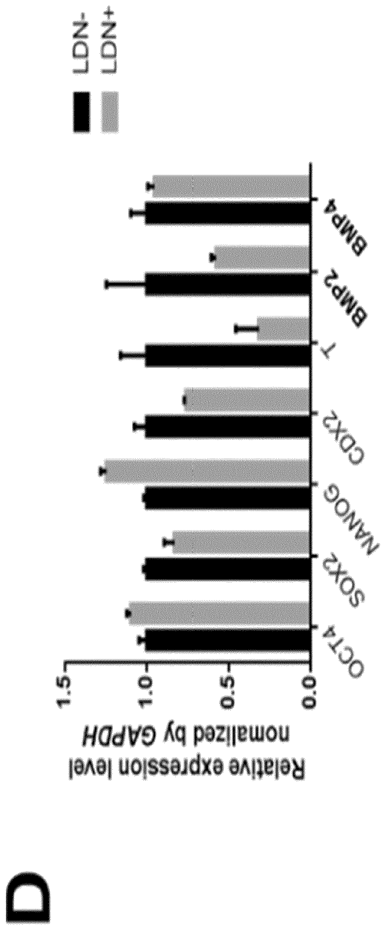

Figure 10 is the results showing the effect of LDN-193189 on porcine embryonic stem cells grown on feeder cells, wherein Figure 10A is the results showing the expression of pluripotency markers (NANOG, SSEA4) in porcine embryonic stem cells grown on feeder cells treated with LDN-193189 (white dotted border: differentiated cell colonies); Figure 10B is the results showing the effect of LDN-193189 on the expression of phosphor-SMAD1/5; Figure 10C shows the results showing the effect of LDN-193189 on the expression of phosphor-SMAD2/3; and Figure 10D shows a result of confirming the expression of genes related to pluripotency and mesodermal tissue development by qPCR in porcine embryonic stem cells grown on feeder cells treated with LDN-193189.

Treatment with LDN-193189 reduced the spontaneous differentiation of porcine embryonic stem cells grown on feeder cells and enhanced the expression of the SSEA4 marker. This is presumed to be because it suppresses the SMAD1/5 signaling system involved in mesodermal differentiation and activates the SMAD2/3 signaling involved in the proliferation of stem cells. In addition, qPCR analysis confirmed that treatment with LDN-193189 reduced the expression of genes involved in the development of various mesodermal tissues.

## Claims

1. A medium composition for culturing porcine pluripotent stem cells, comprising LDN-193189.

2. The medium composition for culturing porcine pluripotent stem cells according to claim 1, wherein the LDN-193189 has a concentration of 10 nM to 1000 nM.

3. The medium composition for culturing porcine pluripotent stem cells according to claim 1, wherein the porcine pluripotent stem cells are porcine embryonic stem cells or porcine induced pluripotent stem cells.

4. The medium composition for culturing porcine pluripotent stem cells according to claim 1, wherein the medium composition for culturing porcine pluripotent stem cells is used for feeder cell-free culture.

5. The medium composition for culturing porcine pluripotent stem cells according to claim 4, wherein the feeder cell-free culture is culturing cells in a culture vessel coated with extracellular matrix (ECM) or Matrigel^{®}.

6. The medium composition for culturing porcine pluripotent stem cells according to claim 5, wherein the feeder cell-free culture is culturing cells in a culture vessel coated with fibronectin, laminin, poly-L-lysine, type 1 collagen or Matrigel^{®}.

7. The medium composition for culturing porcine pluripotent stem cells according to claim 1, wherein the medium composition for culturing porcine pluripotent stem cells further comprises FGF2, activin A, CHIR99021, and IWR-1.

8. The medium composition for culturing porcine pluripotent stem cells according to claim 7, wherein the concentration of FGF2 is 0.1 ng/ml to 100 ng/ml, the concentration of activin A is 0.1 ng/ml to 10 ng/ml, the concentration of CHIR99021 is 0.1 µM to 4.5 µM, and the concentration of IWR-1 is 0.1 µM to 5 µM.

9. The medium composition for culturing porcine pluripotent stem cells according to claim 1, wherein the medium composition for culturing porcine pluripotent stem cells promotes proliferation of stem cells and inhibits apoptosis.

10. The medium composition for culturing porcine pluripotent stem cells according to claim 1, wherein the medium composition for culturing porcine pluripotent stem cells increases the number of stem cell colonies and the size of the colonies.

11. The medium composition for culturing porcine pluripotent stem cells according to claim 1, wherein the medium composition for culturing porcine pluripotent stem cells enhances stemness/pluripotency of stem cells.

12. The medium composition for culturing porcine pluripotent stem cells according to claim 1, wherein the medium composition for culturing porcine pluripotent stem cells comprises 0.1 to 3% by weight of serum-free or serum components.

13. A composition for addition to a porcine pluripotent stem cell culture medium, wherein the composition comprises LDN-193189 as an active ingredient.

14. The composition for addition to a porcine pluripotent stem cell culture medium according to claim 13, wherein the LDN-193189 has a concentration of 10 nM to 1000 nM.

15. A method of culturing porcine pluripotent stem cells, comprising treating porcine pluripotent stem cells with the medium composition for culturing porcine pluripotent stem cells according to claim 1 and culturing them,
wherein the porcine pluripotent stem cells are porcine embryonic stem cells or porcine induced pluripotent stem cells.

16. The method of culturing porcine pluripotent stem cells according to claim 15, wherein the method is a feeder cell-free culture.

17. The method of culturing porcine pluripotent stem cells according to claim 16, wherein the feeder cell-free culture is culturing the cells in a culture vessel coated with extracellular matrix (ECM) or Matrigel.

18. The method of culturing porcine pluripotent stem cells according to claim 17, wherein the feeder cell-free culture is culturing the cells in a culture vessel coated with fibronectin, laminin, poly-L-lysine, type 1 collagen or Matrigel.
